# EUROPEAN PATENT APPLICATION

(11) **EP 2 251 032 A1**
(43) Date of publication of application: **17.11.2010**
(21) Application number: 09159969.6
(22) Date of filing: 12.05.2009
(51) Int. Cl.: A61K 38/40, A23L 1/29, A23L 1/305, A23J 1/20, A23C 9/152, A23C 19/05, A23C 23/00, A61P 25/28

(54) **Lactoferrin and brain health and protection in adults**

(71) Applicant: Nestec S.A., 1800 Vevey (CH)
(72) Inventor: Wang, Bing, 1066 Epalinges (CH); Faure, Magali, 1074 Mollie-Margot (CH); Schmitt, Jeroen Antonius Johannes, 1510 Moudon (CH)
(74) Representative: Marquardt, Ulf

(57) **Abstract**

The present invention relates generally to the field of brain health, brain protection, maintenance of cognitive function, prevention of cognitive decline and cognitive disorders. Neuronal cells in the brain can be protected. Also cognitive performance can be increased.

## Description

The present invention relates generally to the field of brain health, brain protection, maintenance of cognitive function, prevention of cognitive decline and cognitive disorders. Neuronal cells in the brain can be protected. Also cognitive performance can be increased.

In almost every country, the proportion of people aged over 60 years is growing faster than any other age group, which is at least in part due to a longer life expectancy. This population ageing can - consequently - be seen as a success story for the increase in health awareness as well as for the improved availability and performance of public health care. According to the WHO is the world's population of people that are older than 60 years presently 650 million. By 2050, the "greying" population is forecast to reach 2 billion.

Ageing, however, increases the risk top develop some diseases and it remains to be an aim of our society today to allow the population to age in good health. Central to the quality of life - in particular for the ageing population - is a proper cognitive performance and its maintenance.

Most common mental disorders affect cognitive functions, mainly memory processing, perception and problem solving. The most direct cognitive disorders are amnesia, dementia and delirium. Alzheimer's disease (AD) is the most common form of dementia. This presently incurable, degenerative, and terminal disease was first described by Alois Alzheimer in 1906. Generally Alzheimer is diagnosed in people over 65 years of age, although the less-prevalent early-onset Alzheimer's can occur much earlier. An estimated 26.6 million people worldwide had Alzheimer's in 2006; this number may quadruple by 2050. Several age related disorders may be treated or prevented by a proper nutrition. However, with respect to cognitive disorders very little is known about nutritional measures one can undertake to prevent them. There is hence a great need for compositions that may be used to secure a proper cognitive function.

Consequently, it was the object of the present invention to improve the state of the art and in particular to provide a composition that can be used to maintain cognitive function and to prevent cognitive decline and/or cognitive disorders.

The inventors were surprised to see that they could achieve this object by the subject matter of the independent claim.

The present inventors were able to demonstrate that lactoferrin, for example a composition supplemented with lactoferrin, can be used to achieve the object of the present invention.

Lactoferrin (LF), also known as lactotransferrin (LTF), is a globular multifunctional protein that is known to exhibit an antimicrobial activity and is a part of the innate defense, mainly at mucoses.

Lactoferrin may be found for example in milk and whey and in many mucosal secretions such as tears and saliva. As such, Lactoferrin may be purified, e.g., from milk or may be produced recombinantly.

The present invention relates to lactoferrin obtainable from any source.

Lactoferrin from milk or whey for example has the advantage that it is a natural ingredient obtained from a food-grade composition and can consequently be used as enriched fraction of the food composition without further purification.

Recombinantly obtained lactoferrin has the advantage that it can be produced easily in high concentrations.

Human colostrum has a relatively high concentration of lactoferrin, followed by human milk, then cows milk.

Consequently, one embodiment of the present invention is an ingestible composition enriched in lactoferrin.

Enriched means that lactoferrin was either added to the composition, so that the resulting lactoferrin content of the composition is higher than the lactoferrin content of the composition without lactoferrin addition, or that the composition was treated in a way to concentrate the natural lactoferrin content in a composition.

Lactoferrin may also be provided as pure compound.

Alternatively, lactoferrin may be provided as a lactoferrin enriched fraction, for example a lactoferrin enriched milk or whey fraction.

As milk or whey source bovine milk, human milk, goat milk, camel milk, horse milk and/or donkey milk may be used, for example. Colostrum may be used as well.

In therapeutic applications, compositions are administered in an amount sufficient to at least partially cure or arrest the symptoms of the disorder and/or its complications. An amount adequate to accomplish this is defined as "a therapeutically effective dose". Amounts effective for this purpose will depend on a number of factors known to those of skill in the art such as the severity of the disorder and the weight and general state of the patient. In prophylactic applications, compositions according to the invention are administered to a patient susceptible to or otherwise at risk of a particular disorder in an amount that is sufficient to at least partially reduce the risk of developing a disorder. Such an amount is defined to be "a prophylactic effective dose". Again, the precise amounts depend on a number of patient specific factors such as the patient's state of health and weight.

Lactoferrin may be administered in the framework of the present invention in a therapeutically effective dose and/or in a prophylactic effective dose.

Typical lactoferrin enriched compositions may comprise lactoferrin in an amount of at least 1,6 g/L.

For example, the composition of the present invention may contain lactoferrin in a concentration of at least 0.75% (w/w), preferably at least 1% (w/w).

In one embodiment, the composition is to be administered in an amount corresponding to an ingestion of at least 0.25g lactoferrin, preferably at least 0.5 g lactoferrin more preferably at least 1 g lactoferrin per day per kg body weight.

For example, the composition may be consumed in an amount corresponding to at least 1g lactoferrin /kg body weight/day intake for pregnant and/or lactating mothers.

The composition may also be consumed in an amount corresponding to at least 200mg lactoferrin /kg body weight/day intake for the children.

Lactoferrin may be present in the composition in a concentration of at least 0,01 g per 100kcal, preferably of at least 0,1 g per 100kcal. For example, lactoferrin may be present in the composition in the range of about 0,01g -100g, preferably 0,1g - 50 g, even more preferred 2 g - 25 g per 100 kcal of the composition.

Lactoferrin may also be used in combination with other compounds, such as sialic acid and/or iron, for example.

Sialic acid is a generic term for the N- or O-substituted derivatives of neuraminic acid, a monosaccharide with a nine-carbon backbone.

Any sialic acid may be used for the purposes of the present invention. However, it is preferred if the sialic acid has the following formula

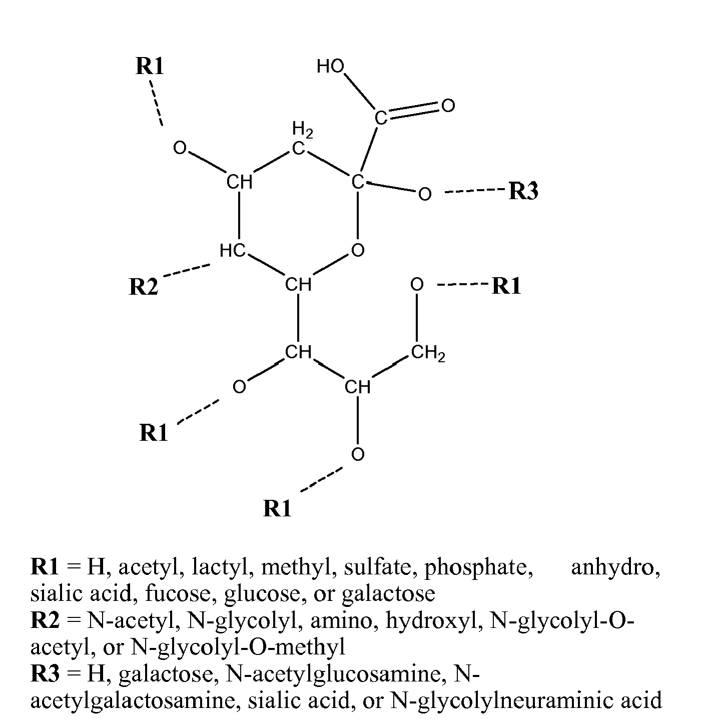

R1 may be selected from the group consisting of H, acetyl, lactyl, methyl, sulfate, phosphate, anhydrosialic acid, fucose, glucose and/or galactose.

R2 may be selected from the group consisting of N-acetyl, N-glycolyl, amino, hydroxyl, N-glycolyl-O-acetyl, and/or N-glycolyl-O-methyl.

R3 may be selected from the group consisting of H, galactose, N-acetylglucosime, N-acetylgalactosamine, sialic acid, and/or n-glycolylneuraminic acid.

The groups in position R1 may be identical or may differ from each other.

For example, the sialic acid may be N-acetylneuraminic acid with R1=H, R2=N-acetyl and R3=H. According to a further embodiment of the present invention the sialic acid may selected from the group consisting of 2-keto-5-acetamido-3,5-dideoxy-d-glycero-d-galactononulosonic acid (Neu5Ac) and 2-keto-3-deoxy-d-glycero-d-galactonononic acid (KDN) or mixtures thereof.

Sialic acid as used in the present invention comprises N-Acetylneuraminic acid, which has the following synonyms and abbreviations: o-Sialic acid; 5-Acetamido-3,5-dideoxy-D-glycero-D-galacto-2-nonulosonic acid; 5-Acetamido-3,5-dideoxy-D-glycero-D-galactonulosonic acid; Aceneuramic acid; N-acetyl-neuraminate; N-Acetylneuraminic acid; NANA, and Neu5Ac.

A particular preferred lactoferrin containing composition may contain additionally sialic acid in an amount in the range of 100 mg/100g (w/w) to 1000 mg/100g (w/w) of the composition, for example in the range of 500 mg/100g (w/w) to 650 mg/100g (w/w) of the composition.

The composition of the present invention may for example comprise at least about 0.001 weight-% sialic acid. In further embodiments of the present invention, the composition may comprise at least about 0.005 weight-%, or at least about 0.01 weight-% of sialic acid.

Alternatively or additionally the lactoferrin containing composition may contain iron in an amount in the range of about 1mg/100g (w/w) to 1g/100g (w/w) of the composition, for example 100 mg/100g (w/w) to 300 mg/100g (w/w) of the composition.

One lactoferrin containing composition may contain for example about 580 mg/100g (w/w) sialic acid and 22 mg/100g (w/w) iron. The lactoferrin containing composition of the present invention may have a caloric density in the range of 30 kcal/100g-1000kcal/100g of the composition, preferably 50 kcal/100g - 450 kcal/100g of the composition. It may for example have a caloric density of about 400 kcal/100g.

The nature of the composition is not particularly limited. It is preferably a composition for oral or enteral administration.

The composition may be for example selected from the group consisting of food products, animal food products, pharmaceutical compositions, nutritional formulations, nutraceuticals, drinks, food additives, and nutritional formulas.

In one typical embodiment of the present invention, the composition will contain a protein source, a lipid source and a carbohydrate source.

For example such a composition may comprise protein in the range of about 1.8 to 6 g/100 kcal, lipids in the range of about 4.4 to 6.5 g/100kcal and/or carbohydrates in the range of about 1.7 to 12 g/100 kcal.

If the composition is liquid, its energy density may be between 60 and 75 kcal/100ml.

If the composition is solid, its energy density may be between 60 and 75 kcal/100g.

The type of protein is not believed to be critical to the present invention. Thus, protein sources based on whey, casein and mixtures thereof may be used, for example. As far as whey proteins are concerned, acid whey or sweet whey or mixtures thereof may be used as well as alpha-lactalbumin and betalactoglobulin in whatever proportions are desired. The whey protein may be modified sweet whey. Sweet whey is a readily available by-product of cheese making and is frequently used in the manufacture of nutritional formulas based on cows' milk. However, sweet whey includes a component which is undesirably rich in threonine and poor in tryptophan called caseino-glycomacropeptide (CGMP). Removal of the CGMP from sweet whey results in a protein with a threonine content closer to that of human milk. This modified sweet whey may then be supplemented with those amino acids in respect of which it has a low content (principally histidine and tryptophan).

A process for removing CGMP from sweet whey is described in EP 880902 and a nutritional formula based on this modified sweet whey is described in WO 01/11990. The proteins may be intact or hydrolysed or a mixture of intact and hydrolysed proteins. It may be desirable to supply partially hydrolysed proteins (degree of hydrolysis between 2 and 20%), for example for subjects believed to be at risk of developing cows' milk allergy. If hydrolysed proteins are required, the hydrolysis process may be carried out as desired and as is known in the art. For example, a whey protein hydrolysate may be prepared by enzymatically hydrolysing the whey fraction in two steps as described in EP 322589. For an extensively hydrolysed protein, the whey proteins may be subjected to triple hydrolysis using Alcalase 2.4L (EC 940459), then Neutrase 0.5L (obtainable from Novo Nordisk Ferment AG) and then pancreatin at 55°C. If the whey fraction used as the starting material is substantially lactose free, it is found that the protein suffers much less lysine blockage during the hydrolysis process. This enables the extent of lysine blockage to be reduced from about 15% by weight of total lysine to less than about 10% by weight of lysine; for example about 7% by weight of lysine which greatly improves the nutritional quality of the protein source.

The compositions of the present invention may contain a carbohydrate source. Any carbohydrate source may be used, such as lactose, saccharose, maltodextrin, starch and mixtures thereof.

The compositions of the present invention may contain a lipid source. The lipid source may be any lipid or. Preferred fat sources include milk fat, palm olein, high oleic sunflower oil and high oleic safflower oil. The essential fatty acids linoleic and α-linolenic acid may also be added as may small amounts of oils containing high quantities of preformed arachidonic acid and docosahexaenoic acid such as fish oils or microbial oils. The lipid source preferably has a ratio of n-6 to n-3 fatty acids of about 5:1 to about 15:1; for example about 8:1 to about 10:1.

The compositions of the present invention may also contain all vitamins and minerals understood to be essential in the daily diet and in nutritionally significant amounts. Minimum requirements have been established for certain vitamins and minerals. Examples of minerals, vitamins and other nutrients optionally present in the composition include vitamin A, vitamin B1, vitamin B2, vitamin B6, vitamin B12, vitamin E, vitamin K, vitamin C, vitamin D, folic acid, inositol, niacin, biotin, pantothenic acid, choline, calcium, phosphorous, iodine, iron, magnesium, copper, zinc, manganese, chloride, potassium, sodium, selenium, chromium, molybdenum, taurine, and L-carnitine. Minerals are usually added in salt form. The presence and amounts of specific minerals and other vitamins will vary depending on the numerous factors, such as age weight and condition of the person or animal the composition is administered to.

The compositions may also comprise at least one probiotic bacterial strain. A probiotic is a microbial cell preparation or components of microbial cells with a beneficial effect on the health or well-being of the host. Suitable probiotic bacterial strains include Lactobacillus rhamnosus ATCC 53103 obtainable from Valio Oy of Finland under the trade mark LGG, Lactobacillus rhamnosus CGMCC 1.3724, Lactobacillus paracasei CNCM I-2116, Lactobacillus reuteri ATCC 55730 and Lactobacillus reuteri DSM 17938 obtainable from BioGaia AB, Bifidobacterium lactis CNCM I-3446 sold inter alia by the Christian Hansen company of Denmark under the trade mark Bb12 and Bifidobacterium longum ATCC BAA-999 sold by Morinaga Milk Industry Co. Ltd. of Japan under the trade mark BB536. The amount of probiotic, if present, likewise preferably varies as a function of the age of the person or animal.

Generally speaking, the probiotic content may increase with increasing age for example from 10³ to 10¹² cfu/g composition, more preferably between 10⁴ and 10⁸ cfu/g composition (dry weight).

The compositions may also contain at least one prebiotic in an amount of 0.3 to 10 weight-%. A prebiotic is a non-digestible food ingredient that beneficially affects the host by selectively stimulating the growth and/or activity of one or a limited number of bacteria in the colon, and thus improves host health. Such ingredients are non-digestible in the sense that they are not broken down and absorbed in the stomach or small intestine and thus pass intact to the colon where they are selectively fermented by the beneficial bacteria. Examples of prebiotics include certain oligosaccharides, such as fructooligosaccharides (FOS) and galactooligosaccharides (GOS). A combination of prebiotics may be used such as 90% GOS with 10% short chain fructo-oligosaccharides such as the product sold under the trade mark Raftilose® or 10% inulin such as the product sold under the trade mark Raftiline®.

A particularly preferred prebiotic is a mixture of galacto-oligosaccharide(s), N-acetylated oligosaccharide(s) and sialylated oligosaccharide(s) in which the N-acetylated oligosaccharide(s) comprise 0.5 to 4.0% of the oligosaccharide mixture, the galacto-oligosaccharide(s) comprise 92.0 to 98.5% of the oligosaccharide mixture and the sialylated oligosaccharide(s) comprise 1.0 to 4.0% of the oligosaccharide mixture. This mixture is hereinafter referred to as "CMOS-GOS". Preferably, a composition for use according to the invention contains from 2.5 to 15.0 wt% CMOS-GOS on a dry matter basis with the proviso that the composition comprises at least 0.02 wt% of an N-acetylated oligosaccharide, at least 2.0 wt% of a galacto-oligosaccharide and at least 0.04 wt% of a sialylated oligosaccharide.

Suitable N-acetylated oligosaccharides include GalNAcα1, 3Galβ1, 4Glc and Galβ1, 6GalNAcα1, 3Galβ1, 4Glc. The N-acetylated oligosaccharides may be prepared by the action of glucosaminidase and/or galactosaminidase on N-acetyl-glucose and/or N-acetyl galactose. Equally, N-acetyl-galactosyl transferases and/or N-acetyl-glycosyl transferases may be used for this purpose. The N-acetylated oligosaccharides may also be produced by fermentation technology using respective enzymes (recombinant or natural) and/or microbial fermentation. In the latter case the microbes may either express their natural enzymes and substrates or may be engineered to produce respective substrates and enzymes. Single microbial cultures or mixed cultures may be used. N-acetylated oligosaccharide formation can be initiated by acceptor substrates starting from any degree of polymerisation (DP) from DP=1 onwards. Another option is the chemical conversion of keto-hexoses (e.g. fructose) either free or bound to an oligosaccharide (e.g. lactulose) into N-acetylhexosamine or an N-acetylhexosamine containing oligosaccharide as described in Wrodnigg, T.M.; Stutz, A.E. (1999) Angew. Chem. Int. Ed. 38:827-828.
Suitable galacto-oligosaccharides include Galβ1, 6Gal, Galβ1, 6Galβ1, 4Glc Galβ1, 6Galβ1, 6Glc, Galβ1, 3Galβ1, 3Glc, Galβ1, 3Galβ1, 4Glc, Galβ1, 6Galβ1, 6Galβ1, 4Glc, Galβ1, 6Galβ1, 3Galβ1, 4Glc Galβ1, 3Galβ1, 6Galβ1, 4Glc, Galβ1, 3Galβ1, 3Galβ1, 4Glc, Galβ1, 4Galβ1, 4Glc and Galβ1, 4Galβ1, 4Galβ1, 4Glc. Synthesised galactooligosaccharides such as Galβ1, 6Galβ1, 4Glc Galβ1, 6Galβ1, 6Glc, Galβ1, 3Galβ1, 4Glc, Galβ1, 6Galβ1, 6Galβ1, 4Glc, Galβ1, 6Galβ1, 3Galβ1, 4Glc and Galβ1, 3Galβ1, 6Galβ1, 4Glc, Galβ1, 4Galβ1, 4Glc and Galβ1, 4Galβ1, 4Galβ1, 4Glc and mixtures thereof are commercially available under the trade marks Vivinal ® and Elix'or ®. Other suppliers of oligosaccharides are Dextra Laboratories, Sigma-Aldrich Chemie GmbH and Kyowa Hakko Kogyo Co., Ltd. Alternatively, specific glycoslytransferases, such as galactosyltransferases may be used to produce neutral oligosaccharides.

Suitable sialylated oligosaccharides include NeuAcα2, 3Galβ1, 4Glc and NeuAcα2, 6Galβ1, 4Glc. These sialylated oligosaccharides may be isolated by chromatographic or filtration technology from a natural source such as animal milks. Alternatively, they may also be produced by biotechnology using specific sialyltransferases either by enzyme based fermentation technology (recombinant or natural enzymes) or by microbial fermentation technology. In the latter case microbes may either express their natural enzymes and substrates or may be engineered to produce respective substrates and enzymes. Single microbial cultures or mixed cultures may be used. Sialyl-oligosaccharide formation can be initiated by acceptor substrates starting from any degree of polymerisation (DP) from DP=1 onwards.

The compositions may optionally contain other substances which may have a beneficial effect such as nucleotides, nucleosides, and the like.

The compositions for use in the invention may be prepared in any suitable manner. For example, a composition may be prepared by blending together the protein source, the carbohydrate source, and the fat source in appropriate proportions. If used, the emulsifiers may be included in the blend. The vitamins and minerals may be added at this point but are usually added later to avoid thermal degradation. Any lipophilic vitamins, emulsifiers and the like may be dissolved into the fat source prior to blending. Water, preferably water which has been subjected to reverse osmosis, may then be mixed in to form a liquid mixture. The liquid mixture may then be thermally treated to reduce bacterial loads. For example, the liquid mixture may be rapidly heated to a temperature in the range of about 80°C to about 110°C for about 5 seconds to about 5 minutes. This may be carried out by steam injection or by heat exchanger; for example a plate heat exchanger. The liquid mixture may then be cooled to about 60°C to about 85°C; for example by flash cooling. The liquid mixture may then be homogenised; for example in two stages at about 7 MPa to about 40 MPa in the first stage and about 2 MPa to about 14 MPa in the second stage. The homogenised mixture may then be further cooled to add any heat sensitive components; such as vitamins and minerals. The pH and solids content of the homogenised mixture is conveniently standardised at this point. The homogenised mixture is transferred to a suitable drying apparatus such as a spray drier or freeze drier and converted to powder. The powder should have a moisture content of less than about 5% by weight. If it is desired to add probiotic(s), they may be cultured according to any suitable method and prepared for addition to the composition by freeze-drying or spray-drying for example. Alternatively, bacterial preparations can be bought from specialist suppliers such as Christian Hansen and Morinaga already prepared in a suitable form for addition to food products. Such bacterial preparations may be added to the powdered composition by dry mixing.

Lactoferrin may be added at any stage during this procedure, but is preferably added after the heating step.

The composition comprises a protein source which may be present in the range of between 1.4 and 100g /100 kcal, preferably between1. 4 and 4.0g/100 kcal of the composition. Since lactoferrin is a protein it should be considered a part of the protein source.

Whey protein is known to provide several health benefits. For example, it is easily digestible. The protein fraction in whey (approximately 10% of the total dry solids within whey) comprises several protein fractions, for example betalactoglobulin, alpha-lactalbumin, bovine serum albumin and immunoglobulins. In one embodiment at least 50%, preferably at least 75 %, even more preferred at least 85 % by weight of the protein source is whey protein.

If present, the lipid source may contribute to between 30 to 55% of the total energy of the composition. A carbohydrate source may contribute to between 35 and 65% of the total energy of the composition.

For the purposes of the present invention it is essential that the composition contains lactoferrin or a compound that yields lactoferrin after consumption. The composition does not have to be enriched in lactoferrin, although this may be preferable, since this way more lactoferrin can be administered in smaller volumes.

The lactoferrin may be used to prepare any kind of composition. It is preferred, however, that the lactoferrin is provided as a composition in accordance with what is described above.

It could be shown that the administration of lactoferrin or of the composition described in the present invention allows it to improve cognitive functioning.

It could further be shown that the administration of lactoferrin or of the composition described in the present invention allows it to increase the neuron density and neuron survival.

Metabolic changes in the hippocampus after lactoferrin administration indicate that learning and short term memory is improved.

Metabolic changes in the cortex after lactoferrin administration indicate that long term memory is improved.

Lactoferrin and the composition described in the present invention were found to be in particular effective when administered to adults and/or to the elderly.

A subject is considered as "elderly" if it has surpassed the first half of its average expected lifespan in its country of origin, preferably, if it has surpassed the first two thirds of the average expected lifespan in its country of origin, more preferably if it has surpassed the first three quarters of the average expected lifespan in its country of origin, most preferred if it has surpassed the first four fifths of the average expected lifespan in its country of origin.

One embodiment of the present invention relates to the use of lactoferrin or lactoferrin enriched compositions for the preparation of a composition for the treatment or prevention of cognitive disorders.

The present inventors have also found that lactoferrin or lactoferrin enriched compositions may be used to treat or prevent neurodegenerative disorders. Lactoferrin is presently thought to promote neuronal survival and repair and/or to limit neuronal oxidative stress and cell death.

Lactoferrin or lactoferrin enriched compositions can furthermore be used to protect neuronal cells against degeneration. Such degeneration may follow, for example, stress. Lactoferrin was found to promote neuronal survival and/or limit or prevent neuronal death. It was also found that lactoferrin and/or the compositions of the present invention may be used to reverse neuronal cell damage and/or neuronal cell degeneration. Hence, the present invention allows it not only to prevent or arrest neuronal cell damage and/or neuronal cell degeneration and disorders associated therewith, it allows also to reverse such conditions.

The present invention hence relates also to Lactoferrin and/or the compositions of the present invention to treat or prevent disorders linked to neuronal cell damage and/or neuronal cell degeneration.

The cognitive disorders that may be treated or prevented by the use of the present invention may be selected from the group consisting of neurodegenerative disorders, cognitive decline, memory loss, sleep disorders, mood disorders, depression and combinations thereof. Typical neurodegenerative disorders are in this respect Alzheimer's disease, Creutzfeldt-Jakob disease, Huntington's disease, Parkinson's disease, and combinations thereof, for example.

The compositions of the present invention may further be used to treat or prevent memory loss, in particular short time memory loss and/or long-time memory loss, an impaired learning ability, an impaired mental performance or a reduced attention span.

Those skilled in the art will understand that they can freely combine all features of the present invention described herein, without departing from the scope of the invention as disclosed. In particular, features described for the uses of the present invention may be applied to composition of the present invention and vice versa.

Further advantages and features of the present invention are apparent from the following Examples and Figures.
Figure 1 shows the percentage of positive NS20Y cells for neurite outgrowth in basal condition (untreated cells) and after treatment of the cells with either the neurotrophic factor CNTF (100 ng / mL, positive control) or the lactoferrin enriched bovine milk fraction at different concentrations. Data are means ± SEM, n=3 to 7 according to the group (Basal, n=7; CNTF, n=3; 1 ug/L, n=3; 10 ug/L, n=7; 100 ug/L, n=3; 1 mg/L, n=3; 10 mg/L, n=5; 100 mg/L, n=7; 1g/L, n=6). Data were compared to the basal untreated group with the student t test. A difference was considered significant when P<0.05.
Figure 2 shows the release of neuron-specific enolase (NSE), a marker for neuronal cell death, by a primary culture of enteric neurons, following H₂O₂ challenge and prevention with bovine milk Lactoferrin. Data are mean ± SEM, n=8. A difference was considered significant when P<0.05
Figure 3 shows the percentage of 7-AAD positive cells in cultured SH-SY5Y cells, following H₂O₂ challenge in presence or not of different concentrations of bovine milk Lactoferrin ranging from 0.001 to 1 g/L.
Figure 4 a shows the brain to body weight ratio at P1 in normal and IUGR rats. The brain to body weight ratio was found to be increased after lactoferrin administration, both in normal rats and even more in IUGR rats.

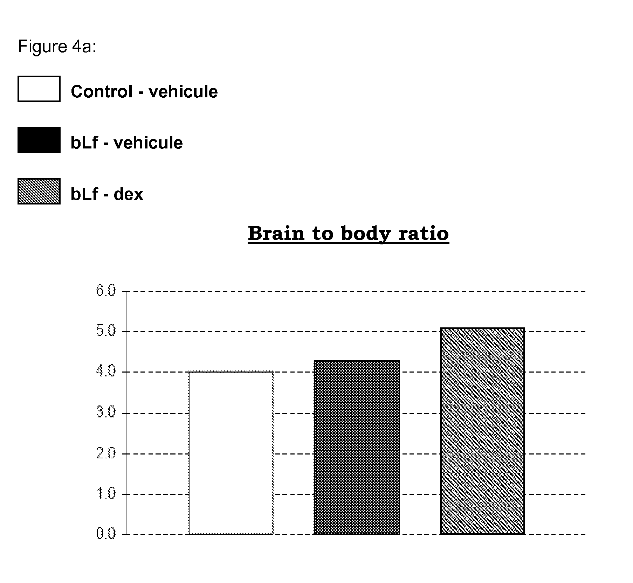

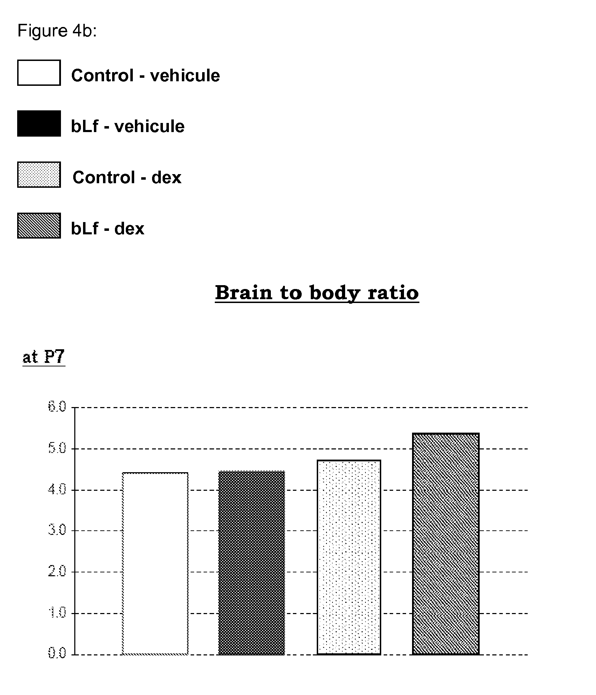

Figure 4 b shows the brain to body weight ratio at P7 in normal and IUGR rats. The brain to body weight ratio was found to be increased after lactoferrin administration, both in normal rats and even more in IUGR rats.

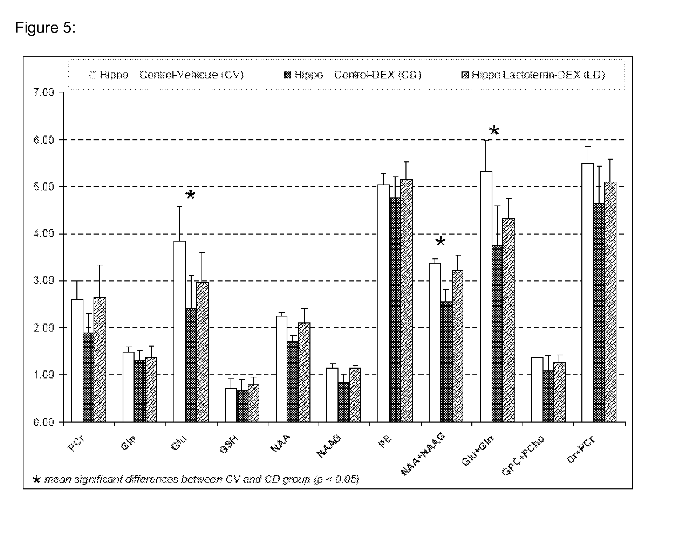

Figure 5 shows the presence of several metabolic markers indicative for brain activity and development in the hippocampus of normal rats, IUGR rats and IUGR rats treated with lactoferrin at P7. Hippocampal activity is linked to learning and short term memory.

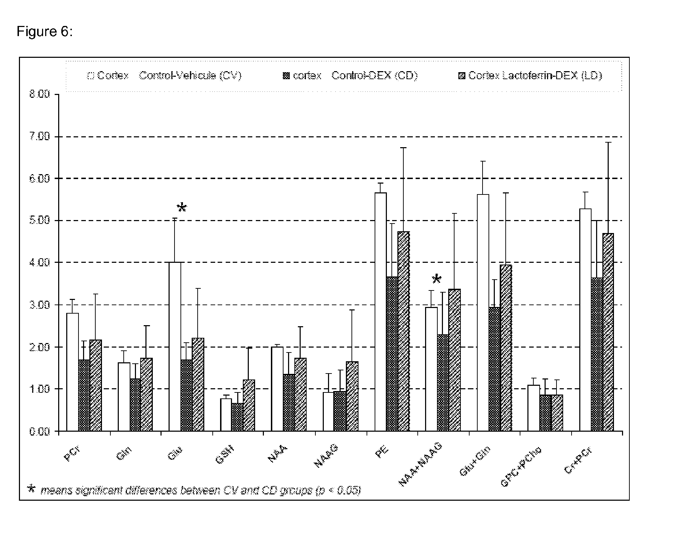

Figure 6 shows the presence of several metabolic markers indicative for brain activity and development in the cortex of normal rats, IUGR rats and IUGR rats treated with lactoferrin at P7. Cortex activity is linked to long term memory.

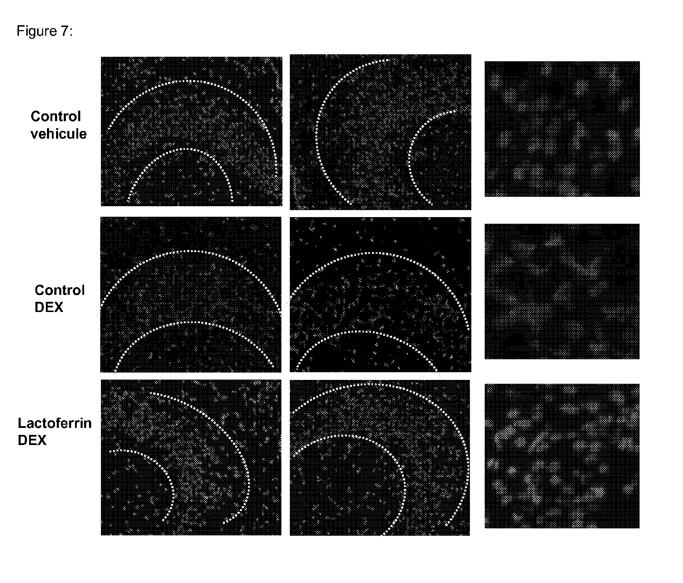

Figure 7 shows the nuclei morphology in the CA2-CA3 field of the hippocampus after DAPI staining.

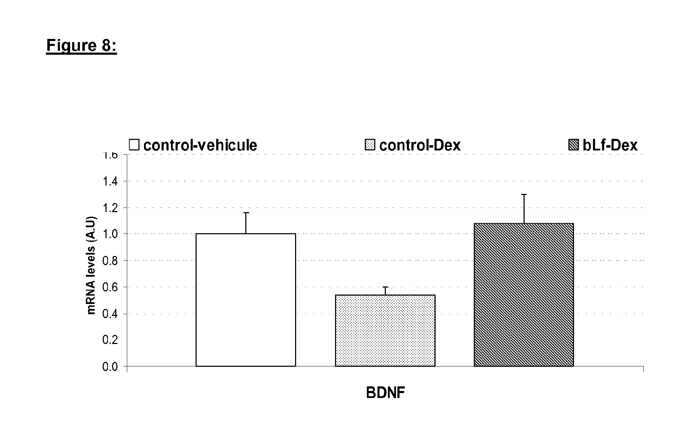

Figure 8 shows that lactoferrin supplementation also increased the gene expression of Brain derived neutrophic factor (BDNF)

Examples:
Biological activity of lactoferrin enriched bovine milk fraction: It has an effect on promoting neuronal cell survival and neurite outgrowth in vitro
The neurite outgrowth process comprises the outgrowth of axons from neurons and is part of neuronal development. The impact of a fraction of bovine milk enriched in lactoferrin on neurite outgrowth was measured using a well established and commonly used in vitro bioassay.

Briefly, NS20Y murine neuroblastoma cells (DSMZ) were thawed from cryogenic storage, plated at a density of approximately 27x10³ cells per cm² in tissue culture-treated flasks (Falcon) and expanded in the presence of DMEM (Gibco) containing 10% FCS (Gibco) and 2 mM L-glutamine (Gibco). Two days after plating, the cells were detached from the flask by mechanical agitation (tapping of the flask), and a single cell suspension was obtained by passing the suspension several times through a flame-polished glass pipette. Cells were then plated onto 13 mm round glass coverslips in the presence of DMEM containing 10% FCS and 2 mM L-glutamine at a density of 2,000 cells per coverslip. The following day the medium was switched to DMEM containing 0.5% FCS, 2 mM L-glutamine, and different concentrations of the milk fractions to be tested. One day later cells were fixed with 4% paraformaldehyde and the coverslips mounted on slides.

All coverslips were imaged with an Axioplan 2 microscope (Zeiss). Digital images were taken from 25 defined fields across the diameter of the coverslip (20X objective, Axiocam MRc, Zeiss). Cells were counted systematically from the first field at the edge of the coverslip across the coverslip until 100 cells had been counted. Cells were scored for either positive or negative for neurite outgrowth. Positive cells for neurite outgrowth were considered if the axon-resembling projections emanating from the cell body reached a length greater than the cell body.

A student t test was used to compare differences in the mean between one control reference population and means from all other treatments in each group.

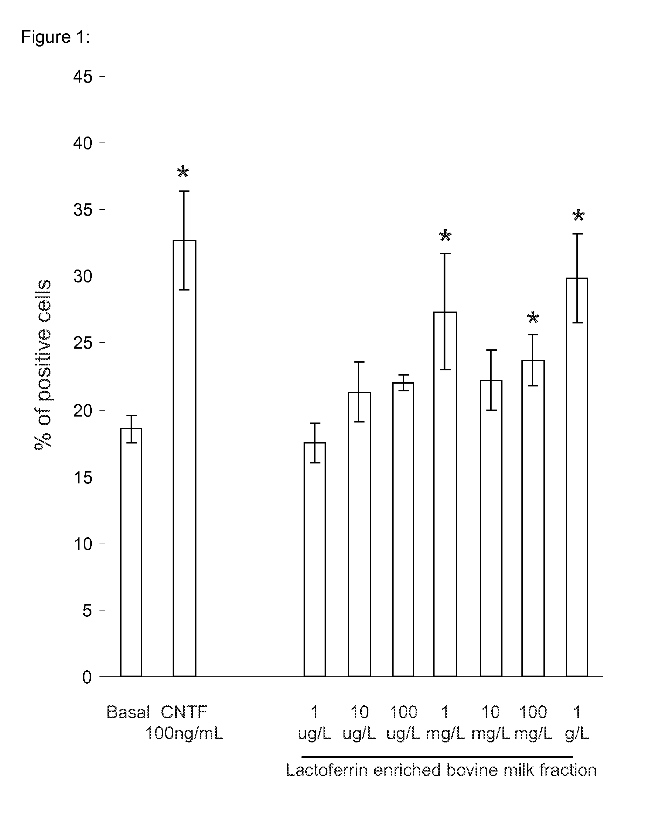

The following concentrations of the lactoferrin enriched bovine milk fraction were tested: 1 µg/L, 10 µg/L, 100 µg/L, 1 mg/L, 10 mg/L, 100 mg/L, and 1 g/L. A positive control (CNTF, ciliary neurotrophic factor, 100 ng/mL), which is a well known neurotrophic factor previously reported to promote neurite outgrowth of different neuronal populations (Oyesiku NM, Wigston DJ: Ciliary neurotrophic factor stimulates neunte outgrowth from spinal cord neu- rons. J Comp Neurol 1996; 364: 68-77.) was performed. A basal control consisted of untreated cells. Results are shown in figure 1.

### Protection of neuronal cells against stress

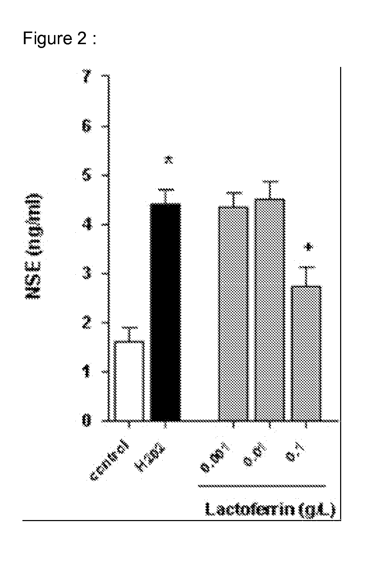

Rat primary cultures of enteric neuronal cells were seeded into wells and incubated with different concentrations of bovine lactoferrin-enriched fraction for 48h. After washing three times with phosphate buffer saline (sterile PBS, 37°C), the cells were incubated for 12 hours in cell medium without lactoferrin and containing H₂O₂ or its vehicle (control). The protective effect of lactoferrin upon H₂O₂-induced neuronal cell death was evaluated by measuring the release of neuron-specific enolase (NSE) in the cell medium. After oxidative stress, the medium of the different groups were collected and centrifuged for 10 min at 12,000 rpm (4°C). The supernatant was collected and the NSE released in the culture medium was quantified by immunoradiometric assay. Results are expressed in ng/mL. As shown on Figure 2, H₂O₂ induced a significant increase of NSE in the medium (p<0.05, n=8). As shown in figure 2, treatment of primary neuronal enteric cells with lactoferrin-enriched fraction significantly prevented the H₂O₂-induced release of NSE (p<0.05, n=8).

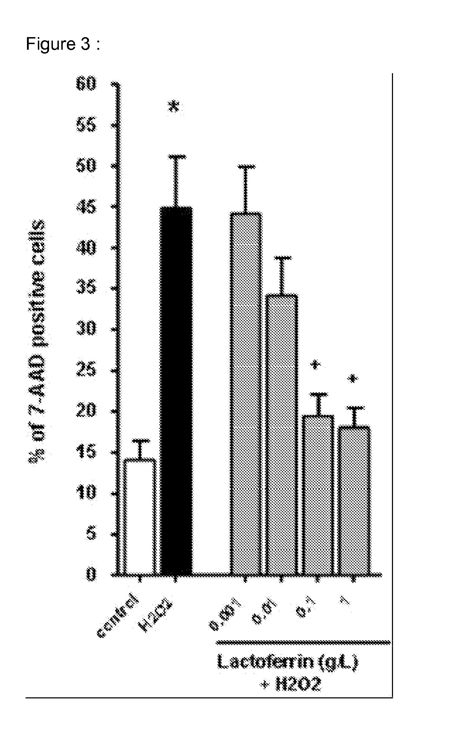

The neuroprotective property of bovine lactoferrin was confirmed using a human neuronal-like cell line (SH-SY5Y-neuroblastoma cells). Briefly, SH-SY5Y cells were plated for 24h, and bovine lactoferrin-enriched fraction was added to the culture media of cells at different concentrations for the following 48h. H₂O₂ for 6h or with its vehicle (control) directly added to the cell media. Cells were finally washed with 0.1 M PBS before being harvested with trypsine-EDTA. Cell suspension was then pooled with the supernatant and centrifuged for 5 min at 2,000 rpm. After centrifugation, the pellet was resuspended in 500 microliter of PBS 0.1M. Membrane permeability was evaluated by flow cytometry using the 7-AAD as fluorescent marker. For this, 200 microliter of cell suspension were incubated with 7-AAD for 10 min before acquisition using BD FACS Array^{™} bioanalyser. This flow cytometric assay using 7-aminoactinomycin D (7-AAD) allowed to distinguish live (7-AAD negative) and late apoptotic/necrotic (7-AAD positive) SH-SY5Y cells in response to oxidative stress. Results as presented in Figure 3 were expressed as percentage of 7-AAD positive cells per total number of cells.
As shown in Figure 3, H₂O₂ induced a significant increase in the percentage of 7-AAD positive cells (p<0.05, n=6). Treatment of SH-SY5Y cells with lactoferrin prevented the H₂O₂-induced increase in percentage of 7-ADD positive cells.

Cell death was strongly increased by the H₂O₂ treatment, reaching about 22%. The preventive treatment of cells with bovine lactoferrin-enriched fraction limited cell death in a dose dependent manner (black bars). Bovine lactoferrin-enriched fraction on its own did not show toxic effect at the concentration tested (grey bars).
Lactoferrin improves the brain/body weight ratio in normal and IUGR rats
Rat model: Wister Rat

Dams are treated with dexamethasone (DEX) during the third week of gestation. This corticosteroid will be delivered during the 3^{rd} of gestation by an osmotic pump Alzet® embeded subcutaneously; sham animals with osmotic pump containing saline buffer will be used as control. This design represents a model of high frailty for pups, mimicking a situation of frailty during the perinatal period in the human species, which is a property model to prove the ability of lactoferrin to enhance the brain development apart from any other interventions. Lactoferrin supplementation will be tested 1) during both gestation and lactation, 2) during lactation and 3) no supplementation. For establishing a logical experimental design allowing proper comparisons, the same supplementation protocol will be applied to normal gestations.

IUGR pups: model of intrauterine growth retardation (IUGR) is obtained by the treatment of dams with dexamethasone (100µg/kg/day) during the third week of gestation. For the nutritional supplementation of gestating dams, lactoferrin will be given orally from the 15^{th} day of gestation to the weaning and food is available *ad libitum.* Lactoferrin will be delivered to newborn rats as earlier as possible until they will be weaned.

The following 6 groups of animals were used:

| | |
|---|---|
| Group 1: | Normal pups; no nutritional intervention in control dams (sham = osmotic pump with saline buffer). |
| Group 2: | IUGR pups; no nutritional intervention in DEX treated dams. |
| Group 3: | Normal pups; bLf supplementation (1g/Kg/day) of control dams (sham) from the beginning of gestation to the end of the lactation. |
| Group 4: | IUGR pups; bLf supplementation (1g/Kg/day) of DEX treated dams from the beginning of gestation to the end of the lactation. |
| Group 5: | IUGR pups; no nutritional intervention in DEX treated dams; vehicule (same volume as bLf) of pups were drop fed 200 mg/kg/day of a blend of amino acids mimicking casein protein in addition to lactation from 1 to 21 days after giving birth. |
| Group 6: | IUGR pups; no nutritional intervention of DEX treated dams; bLf supplementation (200 mg/Kg/day) of pups by drop feeding in addition to lactation from 1 to 21 days after giving birth. |

The results were the following and are shown in Figure 4 a) and b).

The body weight of the offspring at birth of the DEX control and lactoferrin supplementation DEX are about 20~25% smaller than that control vehicule group. This shows that the DEX model is a valid tool to mimick a situation of frailty during the perinatal period in human species.

Thus this model is a property model to demonstrate the ability of lactoferrin to enhance the brain development apart from any other interventions.

Brain weight in both DEX control and DEX lactoferrin supplementation groups were smaller than that control vehicule group. However the decrease of brain weight is smaller than that body weight in Lf supplemented groups, thus brain to body weight ratio is bigger in Dex LF treatment compared to the Dex control group at postnatal day 1.

Interestingly the brain weight of the Dex Lf group caught up to the control vehicule group on postnatal day 21.

### Lactoferrin increases metabolism in the brain

Using LC model analysis, the following 18 metabolites will be quantified from both the cortex and hippocampus: alanine (Ala), aspartate (Asp), creatine (Cr), -aminobutyric acid (GABA), glucose (Glc), glutamate (Glu), glutamine (Gln), glutathione (GSH), glycerophosphorylcholine (GPC), phosphorylcholine (PCho), *myo*-inositol (Ins), lactate (Lac), N-acetylaspartate (NAA), N-acetylaspartylglutamate (NAAG), phosphocreatine (PCr), phosphorylethanolamine (PE), *scyllo*-inositol, and taurine (Tau).

It was aimed to visualize changes in cerebral development following adverse prenatal exposures using *in vivo* MR techniques (use of a 9.4 Tesla scanner at the EPFL), and to assess the effect of early nutritional interventions on brain development and metabolism mainly during the first month of life in our rodent models. Fast Spin-Echo (FSE) images and spectra edition ¹H-MR Spectroscopy were used for the specific local cerebral and hippocampus metabolism. Briefly, FSE images (TR/TE = 6000/80 ms; FOV = 25×25 mm and matrix size = 256×128) were realized to position MRS voxel of interest (VOI = 1.5×1.5×2.5 mm3). First and second order shims were adjusted using FASTMAP [Martin E, 2001, Ann Neurol 49:518-521]. The water linewidths ranged between 8 and 15 Hz. Spectra acquisitions both within the cortical lesion and the contralateral cortical area were performed using an ultrashort echo time (TE/TR = 2.7/4000 ms) SPECIAL spectroscopy method. This method combines 1D image-selected in vivo spectroscopy (ISIS) in the vertical (Y) direction with a slice selective spin echo in the X and Z directions and provides full signal intensity available in the excited region. 35 to 70 series of FIDs (12 averages each) were acquired, individually corrected for frequency drift, summed together and corrected for residual eddy current effects using the reference water signal.

The results were the following and are shown in Figure 5 and Figure 6.

There are significant differences in phosphocreatine (PCr), N-acetylaspartylguatamate (NAAG), N-acetylaspartate (NAA), NAA+NAAG and creatine (Cr) + phosphocreatine (PCr)) concentration between control vehicle (n=5) and control Dex pups (n=4) at 7 days after giving birth (P< 0.05 ~ 0.01). LF treatment Dex pup group (n=6) however, had a trend to reverse the concentrations of above metabolic markers found in Contr-Dex group at P7, but differences did not reach statistic significant in both hippocampus and cortex.

N-Acetylaspartate (NAA), or N-acetylaspartic acid, is a derivative of aspartic acid with a formula of C₆H₉NO₅ and a molecular weight of 175.139. NAA is the second most concentrated molecule in the brain after the amino acid glutamate. NAA is synthesized in neurons from the amino acid aspartate and acetyl coenzyme A. Its proposed primary functions include:
- it is a source of acetate for lipid and myelin synthesis in oligodendrocytes, the glial cells that myelinate neuronal axons
- it is a precursor for the synthesis of the important neuronal dipeptide N-Acetylaspartylglutamate
- it is a neuronal osmolyte that is involved in fluid balance in the brain
- NAA may also be involved in energy production from the amino acid glutamate in neuronal mitochondria

The NAA signal reflects tissue concentrations of both NAA and N-acetylaspartylglutamate (NAAG). NAA has been reported to reflect the presence of neurons, oligodendroglial lineage cells, and axons in the CNS (Urenjak J, 1993, J Neurosci 13:981-989; Martin E, 2001, Ann Neurol 49:518-521; Bjartmar C, 2002, Ann Neurol 51:51-58). It has been suggested that NAA(G) may be an acetyl-group carrier between mitochondria and cytoplasm in neuronal cells (Patel TB, 1979, Biochem J 184:539- 546; Truckenmiller ME, 1985, J Neurochem 45:1658-1662). A decrease of the NAA signal is usually interpreted as a reduction in the number of neurons, but it may also reflect altered function of neuronal mitochondria. The increase of NAA/Cho ratios in cerebral tissue as a result of maturation was previously described in detail and is confirmed in the present study (van der Knaap MS, 1990,Radiology 176:509-515;Kreis R, 2002, Magn Reson Med 48:949-958). N-Acetylaspartylglutamic acid (N-acetylaspartylglutamate or NAAG) is a neuropeptide which is the third-most-prevalent neurotransmitter in the mammalian nervous system. NAAG consists of N-acetylaspartic acid (NAA) and glutamic acid coupled via a peptide bond. NAAG was discovered as a nervous system-specific peptide in 1965 by Curatolo and colleagues (Isaacks RE, 1994, Neurochem Res 19:331-338) but was not extensively studied for nearly 20 years. It meets the criteria for a neurotransmitter, including being concentrated in neurons, packed in synaptic vesicles, released in a calcium-dependent manner, and hydrolyzed in the synaptic space by enzymatic activity. NAAG activates a specific receptor, the metabotropic glutamate receptor type 3. It is synthesized enzymatically from its two precursors and catabolized by NAAG peptidases in the synapse. The inhibition of the latter enzymes has potentially important therapeutic effects in animal models of several neurologic conditions and disorders.

*myo*-Inositol is a crucial constituent of living cells and participates in several physiologic functions. It is a major osmolyte and also serves as the precursor to phosphatidylinositol. *myo*-inositol has been used as a glial cell marker (Isaacks RE, 1994, Neurochem Res 19:331-338). Lac may be used as fuel for the brain but also for the synthesis of myelin (Sanchez-Abarca LI, 2001,Glia 36:321-329).

A decrease of the N-acetylaspartate/choline (NAA/Cho) ratio in asphyxiated full-term neonates predicts an adverse neurodevelopmental outcome (Groenendaal F, 1994,Pediatr Res 35:148-151; Peden CJ, 1993, Dev Med Child Neurol 35:502-510; Roelants-van Rijn AM, 2001, Pediatr Res 49:356-362). Myo-inositol (mI), which is one of the osmoregulators of the brain, can be found in astrocytes and is considered a glial cell marker (Isaacks RE, 1994, Neurochem Res 19:331-338).

Lactoferrin improves neuron density and neuron survival. It also protects from neuronal cell death.

A morphological examination was conducted following MR acquisition. Contiguous sections at the level of the striatum, dorsal and lateral hippocampus were collected to assess cortical and hippocampal architecture and white matter injury. Specific cells types were labeled using immunohistochemistry, in order to determine specific cellular responses. Specific labeling of neurons (NeuN), astrocytes (GFAP) and radial glia (Nestin), in conjunction with markers of white matter myelination (MBP), was performed. The brief methodology was the following:
At P7 and P21, respectively, pups from each group were deeply anesthetized using ketalar (50mg/ml; 0.2-0.5ml, i.p.). Animals were perfused intracardially with 0.9% NaCl, then 4% paraformaldehyde. Brains were removed, weighed and postfixed in 4% paraformaldehyde overnight, then 30% sucrose for 24h minimum, and stored at -80°C until sectioned. Coronal sections (10µm) at the level of the dorsal hippocampus were cut on a cryostat (Microm Cryo-Star HM 560M, Microm International, Germany). Three sections at 200 µm intervals were collected from each animal.

Immunohistochemitry: Brain tissue was processed for immunoreactivity to MBP (1:400 brand city country) using the avidin-biotin peroxidase complex (ABC; Vector Laboratories, Burlingame, CA, USA). Sections were blocked in 4% bovine serum albumin (BSA brand city country), then incubated with the primary antibody for 24h at 4°C, after which they were incubated with the secondary antibody (1:200 brand city country), then with the avidin-biotin complex (1:200, Vector Laboratories, Burlingame, CA, USA). Sections were reacted with the chromagen, 3,3-diaminobenzidine (DAB brand city country) in 0.01% hydrogen peroxide, then coverslipped.

The same protocol was used for fluorescence immunohistochemistry for nestin (1:500 brand city country), GFAP (1:400 brand city country), and NeuN (1:200 brand city country), except that sections were not incubated in the avidin-biotin complex and DAB.

Each experimental group and their respective controls were stained simultaneously. When the primary antibody treatment was omitted, staining failed to occur.

Quantitative analyses were performed using MetaMorph® Imaging System (Meta Imaging Software, Molecular Devices Corporation, Pennsylvania, U.S.A). Values for each animal were pooled and a mean of means ± SEM was calculated for each group. Measurements were made on coded slides blinded to the observer with the codes not being disclosed until the conclusion of analyses.

The results were the following and are shown in Figure 7.

The histological analysis revealed that LF supplementation Dex pup (n=5) has significant increased the Nuclei morphology and neuron density in the CA2-CA3 field of the hippocampus compared to the Dex control pup at P7. A decrease in neuronal density in the cortex at P7 suggests neuronal loss. The neuronal density is similar to the normal control vehicle group (Figure 7), showing that lactoferrin administration can reverse neuronal cell loss.

Lactoferrin supplementation also increased the gene expression of Brain derived neutrophic factor (BDNF) at postnatal day 7 (Figure 8).

BDNF is a neurotrophic factor that promotes neuronal differentiation, survival, and plasticity in the peripheral nervous system and central nervous system (CNS). In CNS, BDNF elicits long-term potentiation, which is related to synaptic plasticity. BDNF promotes neurogenesis. Glutamate also stimulates the production of BDNF (in MRS seems increased by bLF).

BDNF is a key molecule involved in many neuronal aspects of developing and mature neurons. In particular, BDNF promotes the outgrowth of neurites and increases the expression of synaptic proteins, which are required for establishing synaptic connections or functions during development. These findings confirm that dietary lactoferin promotes neurodevelopment and provides neuroprotection.

Hence dietary lactoferrin will influence neuronal density in the hippocampus, an area of great vulnerability for undernutrition and stress related brain abnormalities,implying that LF administration promotes neuron generation and increases neuron survival and neuron protection.

## Claims

1. Ingestible composition enriched in lactoferrin.

2. Composition in accordance with claim 1, wherein the composition is selected from the group consisting of food products, animal food products, pharmaceutical compositions, nutritional formulations, nutraceuticals, drinks, food additives, nutritional formulas.

3. Composition according to any preceding claim, wherein the lactoferrin is provided as a milk or whey fraction enriched in lactoferrin.

4. Composition according to any preceding claim, comprising a source of protein, a source of lipids, a source of carbohydrate, and lactoferrin in a concentration of at least 0.1g/100 kcal of the composition.

5. Composition according to any preceding claim, wherein the source of protein is present in an amount of between 4 and 6.0g/100 calories of the composition.

6. Composition according to any preceding claim, wherein over 50% by weight of the protein source is whey protein.

7. Composition according to any preceding claim wherein the lipid source contributes to between 30 to 55% of the total energy of the composition and/or the carbohydrate source contributes to between 35 and 65% of the total energy of the composition.

8. Composition according to any preceding claim, wherein the lactoferrin is present in a concentration in the range of about 0,01g -100g, preferably 0,1g - 50 g, even more preferred 2 g - 25 g per 100 kcal.

9. Composition according to any preceding claim, further comprising at least about 70 mg/L of total sialic acid, at least about 0.1 % omega-3 fatty acid by weight of total fatty acids, and/or at least about 0.25% omega-6 fatty acids by weight of the total fatty acids.

10. Composition according to any preceding claim, wherein the composition is to be administered in an amount corresponding to an ingestion of at least 0.01g lactoferrin per kg body weight per day.

11. Lactoferrin or a composition in accordance with one of the preceding claims for the treatment or prevention of cognitive disorders.

12. Lactoferrin or a composition in accordance with one of the preceding claims for the repair of neuronal cell damage and/or to promote longevity of neuronal cell.

13. Lactoferrin or a composition in accordance with claim 11, wherein the cognitive disorder is selected from the group consisting of neurodegenerative disorders, cognitive decline, memory loss, sleep disorders, mood disorders and/or depression and combinations thereof.

14. Lactoferrin or a composition in accordance with claim 13, wherein the neurodegenerative disorder is selected from the group consisting of Alzheimer's disease, Creutzfeldt-Jakob disease, Huntington's disease, Parkinson's disease, and combinations thereof.

15. Lactoferrin or a composition in accordance with one of the preceding claims to be administered to adults or the elderly.
